Europäisches Patentamt

European Patent Office

Office européen des brevets

(11) Publication number: **0 617 127 A1**

# EUROPEAN PATENT APPLICATION

(21) Application number: 94103256.7

(22) Date of filing: **29.12.88**

(51) Int. Cl.⁵: **C12N 15/13**, C07K 15/00, C12P 21/08, A61K 39/395, C12N 15/06, G01N 33/577

This application was filed on 04 - 03 - 1994 as a divisional application to the application mentioned under INID code 60.

(30) Priority: **31.12.87 US 140036**
**29.07.88 US 226421**
**05.08.88 US 229178**
**16.11.88 US 272243**
**28.12.88 US 291068**

(43) Date of publication of application:
**28.09.94 Bulletin 94/39**

(60) Publication number of the earlier application in accordance with Art.76 EPC: **0 407 392**

(84) Designated Contracting States:
**AT BE CH DE FR GB IT LI LU NL SE**

(71) Applicant: **TANOX BIOSYSTEMS, INC.**
**10301 Stella Link Rd.**
**Houston, TX 77025 (US)**

(72) Inventor: **Chang, Tse-Wen**
**3323 Robinhood**
**Houston, TX 77005 (US)**
Inventor: **Sun, Bill, Nai-Chau**
**3103 Nottingham Boulevard**
**Houston, TX 77005 (US)**
Inventor: **Sun, Cecily, Rou-Yun,**
**3103 Nottingham Boulevard**
**Houston, TX 77005 (US)**

(74) Representative: **VOSSIUS & PARTNER**
**Postfach 86 07 67**
**D-81634 München (DE)**

(54) **Antigenic epitopes on B cell-bound IgE Immunoglobulins.**

(57) Unique antigenic epitopes of IgE (designated *ige.bl*) which are present on IgE-bearing B lymphocytes but not basophils are described. One class of *ige.bl* epitopes are at or near the FcεR binding site on IgE. Another class of *ige.bl* epitopes are associated with the extracellular segment of the domain of ε chains which anchor IgE to the B cell membrane (ε.mb/ec)).

EP 0 617 127 A1

Background

The immediate-type hypersensitivity, such as extrinsic asthma, hay fever, and allergic responses to certain food or drugs, is mediated primarily by immunoglobulin E (IgE). In an IgE-mediated allergic response, the allergen binds to IgE on the surface of mast cells and basophilic leukocytes (basophils). This binding causes a crosslinking of the IgE molecules and hence the underlying receptors for the Fc portion of IgE (FcεR) and thereby triggers the release of pharmacologic mediators such as histamine, slow-reacting substance of anaphylaxis and serotonin. The release of these mast cell and basophil products causes the various pathological manifestations of allergy.

Patients affected with IgE-mediated hypersensitivity reactions are often treated with histamine antagonists to alleviate symptoms. In addition, during hay fever seasons, desensitization procedures are used to prevent or to alleviate persistent, lasting allergic reactions. In these procedures, small doses of allergen are injected periodically to induce certain, not-fully-understood immune responses that somehow reduce the IgE-mediated responses. Densensitization procedures have been effective in certain patients and only marginally effective in others.

It has been suggested that IgE-mediated allergy might be treated by inhibiting the binding of IgE to mast cells and basophils. For example, synthetic peptides representing various regions of the Fc of IgE (Fcε)have been explored as competitive inhibitors for the binding of IgE to the receptors on mast cells and basophils. See e.g., Stanworth, D.R., Molec. Immun. 21:1183-1190 (1984); Stanworth, D.R. and Burt, D.S., Molec. Immun. 23:1231-1235 (1986); Burt, D.S. et al., Molec. Immun. 24:379-389 (1987); Hahn, U.S. Patent No. 4,683,292; Hamburger, U.S. Patent Nos. 4,171,299 and 4,161,522. However, presumably due to the much lower affinity of these peptides compared with whole IgE for the FcεR, such peptides have not been proven highly efficacious for treatment of allergy.

In recent years, monoclonal antibody methodologies have been employed to map the various antigen and functional epitopes on IgE. Baniyash and Eshhar (Eur. J. Immunol. 14:799-807 (1984)) reported that among the several rat monoclonal antibodies made against IgE, three inhibited the binding of mouse IgE to rat basophilic cells. Since the antibodies could also induce serotonin release from basophils bound with IgE, the antibodies probably bound sites on Fc, which were near but not in the site binding to the receptors for IgE on basophils. More recently the same investigators developed a monoclonal antibody that could inhibit the binding of the IgE to basophilic cells but does not recognize IgE on basophil surface. Baniyash et al., Molec. Immunol. 25:705 (1988). Siraganian and colleagues (see Fed. Proc. 40:965 (1981), Fed. Proc. 46:1346 (1987)) reported that among approximately ten mouse monoclonal antibodies made against human IgE, a few could not bind IgE on basophils. They also described that some of these latter monoclonal antibodies could inhibit the binding of human IgE to basophils. These studies have addressed the use of monoclonal antibodies to define the various epitopes or functionally related peptidic segments on IgE.

Recently, Whitaker described an immunotoxin specific for the IgE isotype and its use in the treatment of allergy. U.S. Patent 4,714,759. The immunotoxin comprises an anti-IgE antibody coupled to a toxin. The intended pharmacologic mechanism of the treatment is that the immunotoxin specific for IgE isotype would kill IgE-producing B cells.

Summary of the Invention

This invention pertains to the unique antigenic determinants on IgE molecules and to reagents and methods of treating IgE-mediated allergy based on the discovery of these determinants. The antigenic determinants are present on IgE-bearing B-lymphocytes (B cells) but not on basophils and mast cells.

Although IgE is produced by only IgE-bearing B cells, it is present not only on these cells but also on mast cells and basophils. IgE has a very high affinity for the FcεR on the surface of basophils and mast cells (the association constant, Ka, is in the range of $10^9$-$10^{10}$ liter mole$^{-1}$) and the rate of dissociation is very slow (the half life of "on time" is about 20 hours). Thus, IgE is virtually a surface antigen of basophils and mast cells.

The antigenic epitopes on IgE of this invention are present on IgE-bearing B cells but not on basophils or mast cells and, because of this, the epitopes are unique surface markers of IgE-bearing B cells. The epitopes can be designated ige.bl (bl depicts B lymphocytes). The IgE on B cells is the membrane-bound form that is anchored on the membrane by spanning through the membrane lipid bilayer; the IgE on basophils and mast cells is the secretory form that is anchored on the cell surface by binding to the FcεR molecules. The overall structures of the two forms of IgE are somewhat different with the membrane-bound form having an extra segment. The topography of association with the cell surface is also different between the IgE on B cells and on basophils. One class of ige.bl epitopes are located in the Fc region of the IgE molecule at or near the binding site of FcεR. These epitopes are obscured by FcεR binding. Another class of ige.bl epitopes are antigenic epitopes that encompass parts of the extracellular segment of membrane-bound region of immunoglobulin ε heavy chains. In general, these segments of various membrane-bound immunoglobulin can be designated mb/ec. The mb/ec segment of IgE can be designated ε.mb/ec segment. The present invention pertains to the sequence determination of ε.mb/ec segment.

The identification of the ige.bl epitopes provides for various forms therapy and diagnosis of IgE-mediated allergy based upon agents which specifically bind to the epitopes. These include allergic diseases such as extrinsic bronchial asthma, allergic rhinitis or hay fever, and food and drug allergies. The preferred agents which specifically bind to the epitopes are monoclonal antibodies or fragments thereof. However, for purposes of the therapeutic methods of this invention, the specific binding agent can be any molecule which specifically binds to the epitopes (and thus binds IgE-bearing B cells but not basophils). These reagents include synthetic peptides which are indentical or analogous to the antigen binding region (variable of hypervariable regions) of anti-ige.bl antibodies. Although the description below focuses mainly on anti-ige.bl antibodies, the concept and methodologies are equally applicable to other molecules specific for the unique epitopes described herein.

Monoclonal antibodies specific for an ige.bl epitope can be used to selectively destroy IgE-producing cells. Because the epitope is present on IgE-bearing B cells and not on mast cells or basophils, monoclonal antibodies specific for an ige.bl epitope bind B cells, but not mast cells or basophils. This differential binding allows the targeting and selective elimination of IgE-producing B cells. The antibodies, either free or in toxin-conjugated form, can be used to target and eliminate the B cells. The monoclonal antibodies, which are specific for the ige.bl epitope at or near the FcεR site of binding to IgE, may have additional therapeutical effects. The antibodies bind IgE forming immune complexes, facilitating the removal of IgE from the immune system by the retiendothelial system.

This invention also pertains to paratope-specific, anti-idiotypic antibodies of the antibodies reactive with ige.bl epitopes, to peptides which are modeled after the epitopes (e.g., the site of FcεR binding) and to the use of the anti-idiotypic antibodies and the peptides to treat IgE-mediated allergic diseases.

Brief Description of the Figures

Figure 1 outlines the process of determining mb/ec segment of human IgE (ε.mb/ec).
Figure 2 shows DNA probes to be used for screening cDNA library to clones containing ε.mb/ec.
Figure 3 shows the C-terminus of CH4 domain of human ε-chain and the locations of probe b and d.

Detailed Description of the Invention

1. Unique Epitopes of Immunoglobulin E

This invention is based on the discovery of uniquely situated epitopes of IgE (ige.bl). The epitopes are present on IgE-bearing B lymphocytes but not on mast cells and basophils. One class of ige.bl epitopes is located at or near the binding site for FcεR. The presence of these epitopes only on IgE-bearing B cells follows from the fact that the epitope is obscured when IgE is bound to FcεR on basophils and mast cells. The ige.bl epitopes may possibly be composed of several antigenically discrete sites and each of them can be defined by its reactivity with a monoclonal antibody which reacts with human IgE-bearing B cells, but not basophils, such as E8-5-3, E11-4-70, E101-1, and E10-12-55 (see below).

Another class of ige.bl epitopes are the ε.mb/ec epitopes. These epitopes are present on the membrane-bound form of immunoglobulin but not on the secreted form. B cells express on their surface antibody molecules such as IgE which serve as receptors for antigens during immunological induction. The membrane-bound immunoglobulins differ from the secretory, soluble immunoglobulins synthesized by the same cells in that they have an extra peptidic piece that anchors them onto the B cell surface. All the ten membrane-bound immunoglobulins on B cells from different species, for which amino acid sequences have been determined, have extra isotype-specific regions that anchor the immunoglobulins to the membrane. These peptidic regions have lengths ranging from 41 to 72 amino acids and can be divided into three segments in terms of locations in relation to the plasma membrane. The middle segments of 25 hydrophobic and uncharged amino acid residues are in the membrane lipid bilayer; the C-terminal hydrophilic segments of 3-28 amino acid residues are intracellular; the segments toward N-terminus contain 13 to 27 amino acid residues, are highly acidic and hydrophilic and are on the extracellular surface of the plasma membrane. This part of membrane-bound region of mouse and rat IgE has 19 amino acid residues, among them, 10 are Glu or Asp residues. The length and the hydrophilic and highly charged nature of the extracellular segment indicate that this segment is exposed and accessible to antibodies.

Because IgE is present on the surface of only three cell types in the body, IgE-bearing B cells, basophils, and mast cells, these ige.bl epitopes are virtually unique cell surface marker of IgE-bearing B cells. These new markers provide for several types of monoclonal-antibody-based therapy of IgE-mediated allergic diseases.

## 2. Monoclonal Antibodies Which Bind IgE-Bearing B Cells but not Basophils

Monoclonal antibodies specific for the ige.bl epitopes bind IgE on the surface of IgE-producing B cells but do not bind basophils. This differential binding of IgE-bearing cell types provides a basis for therapeutic and diagnostic uses for the antibodies.

It is crucial that the antibodies do not bind basophils and induce histamine release. One of the most powerful agents that trigger the release of pharmacological mediators of allergy from mast cells and basophils is anti-IgE antibody. Conventional anti-IgE antibody will bind IgE on the surface of mast cells and basophils and trigger the release of pharmacological mediators of allergy. The antibodies of this invention cannot bind IgE on these cells because the cognate epitope is masked and thus they do not induce the release of histamine from these cells.

## 3. Therapy of IgE-mediated Allergy based upon the Selective Elimination of IgE-producing Cells.

### A. Antibodies specific for IgE-producing cells.

The antibodies specific for IgE-producing B cells, as murine, human, or mouse/human chimeric antibody, may be applied in several ways for the treatment of IgE-mediated allergies. For this purpose, the antibodies can be used to selectively deplete IgE-bearing B cells. The antibodies can be used as an effector agents mediating an immune function or as a carrier agent of toxins or cytotoxic drugs, as set forth below, for delivering an effector substance.

Among the various immune functions that an antibody specific for a surface antigen on target cells can mediate are antibody and complement-mediated cytolysis and antibody-dependent cellular cytotoxicity (ADCC). The antibody may also mediate immune regulatory functions. Antibodies of certain IgG subclasses, such as mouse IgG2a and human IgG1 and IgG3, can mediate ADCC carried out by certain Fc receptor-bearing phagocytic leukocytes. For example, OKT3, a mouse IgG2a monoclonal antibody specific for human T cell surface antigen (the first monoclonal antibody approved by FDA for marketing as a therapeutical agent) is used in patients to provide rapid depletion of T cells in the blood and to induce an immunosuppressed state (for kidney transplantation). Russell, P.S. et al., Annu. Rev. Med. 35:63-79 (1984); Norman, D.J. et al., Transpl. Proc. 17:39-41 (1985). OKT3, at a dosage of 5mg/day/subject, can deplete completely circulating T cells. The antibodies of this invention, especially in the form of mouse gamma 2a antibodies or chimeric antibodies bearing human gamma-1 or gamma-3 chains, can be used to deplete IgE-bearing B cells by the ADCC mechanism. The antibodies can be administered as free antibodies to patients afflicted with IgE-mediated allergy in amounts sufficient to eliminate substantially IgE-producing cells and consequently, to deplete substantially IgE. For example, the amount can range from 1-50mg/dose/subject. The treatment may involve one or several injections. The duration of the presence of antibody and the amount of antibody needed to eliminate B cells is less than that necessary to continuously inhibit the binding of IgE to basophils and mast cells.

For therapeutic uses described, human antibodies are most preferred. Human antibodies, however, are difficult to produce in large quantity. As an alternative, chimeric or "near-human" antibodies are preferred. Chimeric antibodies comprise a variable or antigen binding (hypervariable or complementarity determining) region derived from an animal (e.g., a mouse) antibody and the remaining regions derived from a human antibody. Methods for producing chimeric (e.g. murine/human) antibodies are described below. Chimeric antibodies can be produced in large quantities and they are less immunogenic in humans than nonhuman antibodies. Consequently, they are better suited for in vivo administration than animal antibodies, especially when repeated or long term administration is necessary. Antibody fragments of the chimeric antibodies can also be used.

Immunotherapies employing the antibodies of this invention may be used in combination with conventional desensitization immunotherapy. For example, desensitization with allergen may be performed in conjunction with the administration of anti-ige.bl antibodies or immunotoxins (see B section below) to eliminate substantially IgE-producing cells. One major effect of desensitization is that IgG's are induced against the allergen/immunogen. The induction of an IgG response may be most effective when IgE-producing B cells are substantially depleted. The combination of antibody and desensitization therapy is an attractive form of therapy. IgE-producing B cells may be temporarily depleted (for a few weeks or months) by the anti-ige.bl antibody and will eventually repopulate. The desensitization may have longer lasting effects.

B. Immunotherapy combining an ige.bl-specific antibody and a factor enhancing ADCC.

Many factors, such as GM-CSF (granulocyte monocyte-colony stimulation factor) or M-CSF (monocyte-colony stimulation factor), are known to induce the proliferation of leukocytes, including those mediating ADCC. In in vitro experiments, GM-CSF and M-CSF have been shown to augment the ADCC activity on tumor cells mediated by monoclonal antibodies specific for surface antigens expressed on the tumor cells. It is conceivable that the therapeutical effect of ige.bl specific monoclonal antibodies in treating allergies can be enhanced by combining the use of factors that augment ADCC activities.

C. Immunotoxins specific for IgE-producing cells.

The ige.bl epitopes provide highly specific targets for immunotoxins directed against IgE-producing B cells. Immunotoxins specific for the epitopes bind to IgE-producing B cells but not to mast cells or basophils. In this way, IgE-producing B cells can be selectively reduced or eliminated in a patient suffering from an IgE-mediated allergy. The reduction of the IgE producing cells reduces IgE levels in the circulation which results in a reduction of the amount of IgE available to bind mast cells and basophils. The immunotoxin does not kill mast cells or basophils and cause the release of pharmacologic mediators from these cells.

Immunotoxins for selective binding to IgE-producing lymphocytes are comprised of cytolytic or cytotoxic agents conjugated to a binding agent specific for an ige.bl epitope. The preferred specific binding agents are anti-ige.bl antibodies or fragments thereof (e.g., $F(ab)'_2$, Fab, $F_v$ or analogues or derivatives thereof). The cytolytic agents can be selected from any of the available substances including ricin, Pseudomonas toxin, diptheria toxin, pokeweed antiviral peptide, trichothecenes, radioactive nuclides, and membrane-lytic enzymes. The antibody and the cytotoxin can be conjugated by chemical or by genetic engineering techniques.

The immunotoxins are administered to a patient afflicted with IgE-mediated allergy in amounts sufficient to reduce or to eliminate IgE-producing lymphocytes in the patient and thereby prevent or alleviate the symptoms of the IgE-mediated allergy. The immunotoxins may be used alone or in combination with free anti-IgE antibody.

4. Removal of IgE by Monoclonal Antibodies Which Do Not Bind IgE on Basophils and Mast Cells.

In certain embodiments, the monoclonal anti-IgE antibodies of this invention bind to an ige.bl epitope located at or near the FcεR binding site on soluble IgE. In so doing, the antibodies block the binding site on IgE for mast cells and basophils and this contributes partly to their therapeutical efficacy. In order to achieve this possibly beneficial additional therapeutic mechanism, the antibodies must bind specifically to the epitope with an affinity which is greater than the affinity with which the FcεR binds IgE. This combination of specificity and affinity provides for effective inhibition of IgE binding to FcεR at pharmaceutically feasible concentration. In addition, the immune complexes of IgE and the monoclonal antibodies may be removed rapidly by cells of the reticuloendothelial system. Upon the intravenous injection of the monoclonal antibodies, most IgE molecules in the circulation probably form immune complexes and are removed rapidly. These mechanisms will reduce the levels of free IgE in the body of the treated patient, whenever the therapeutic monoclonal antibodies are present in sufficient levels. These various mechanisms should contribute to the short term effects of the therapeutic antibodies. However, long-term therapeutic effects probably can be achieved without these mechanisms. Because IgE turns over and regenerates rapidly with a serum half life of 3 days, an ige.bl antibody-based therapy probably will be most efficacious by depleting or reducing IgE-producing B cells.

Affinity can be represented by the affinity or association constant, Ka. The FcεR of mast cells and basophils has an estimated Ka in the range of $1 \times 10^9$ - $1 \times 10^{10}$ liter/mole. See Kulczycki, A., New Trends in Allergy II ed. by Ring, J. and Burg, G., pp. 25-32, Springer-Verlag, New York, 1986. Thus, a monoclonal anti-IgE antibody will effectively compete with FcεR, if its affinity constant for IgE is about or greater than $1 \times 10^9$ liter /mole. In general, monoclonal antibodies have, Ka's, below $1 \times 10^9$ liter/mole. Consequently, although an antibody might be appropriately specific for the epitope(s) described and suitable for eliminating IgE-bearing B cells by mechanisms described above, it may not have this additional therapeutical effect, if it does not have a Ka sufficient to compete effectively with FcεR for IgE at pharmacologically feasible concentrations.

In preferred embodiments, the monoclonal anti-IgE antibodies of this invention have a Ka for IgE which is comparable or greater than the affinity of FcεR for IgE. Mast cells and basophils in the body are bound with IgE; in most individuals a large proportion of the FcεR are saturated by IgE. The IgE molecules have very long half lives of "on" (receptor occupancy) time of about 20 hours. Kulczycki, A., J. Exp. Med. 140:1976 (1974). When the IgE molecules dissociate from the mast cells or basophils, the high affinity anti-ige.bl antibodies administered to the patient will tightly bind them and inhibit their reassociation with FcεR. Because IgE molecules have a long "on" time and dissociate slowly from the basophils and mast cells, the therapeutic effects probably will be attained only a few days after the treatment (or even longer) when a significant portion of the IgE on basophils and mast cells has dissociated from FcεR and has been complexed or cleared away by the anti-IgE antibody. When this is achieved, the density of IgE molecules bound to FcεR molecules on basophils and mast cells is substantially decreased and consequently, allergens are unable to cross-link sufficient numbers of FcεR to induce histamine.

### 5. Diagnostic Uses of Antibodies which Specifically Bind IgE-producing Cells.

The antibodies against ige.bl epitopes can be used to identify and enumerate IgE-bearing lymphocytes in mixed leukocyte populations. For this purpose, the antibodies can be used in standard assay formats for determining cell surface antigens. In general, the antibody is contacted with a sample of the leukocytes to be tested under conditions which allow the antibody to bind IgE-bearing cells in the sample. The cells are then examined for binding of antibody. This can be accomplished by conventional cell staining procedures. For example, a fluorescently labeled second antibody can be used to detect binding of the anti-IgE antibody.

### 6. Antiidiotypic Antibodies and Methods of Active Immunization Against IgE.

The ige.bl-specific monoclonal antibodies described thus far can be used to generate paratope-specific, anti-idiotypic antibodies which offer another mode of treating IgE-mediated allergy. Antibodies against the paratope of the ige.bl-specific antibodies conformationally resemble the epitope for which the anti-IgE antibody is specific, that is, they resemble an ige.bl epitope. These anti-idiotypic antibodies can be used to actively immunize against ige.bl and induce the endogenous formation of antibodies against the ige.bl epitope. The induced antibodies will mediate the various therapeutical effects of ige.bl-specific antibodies.

Because IgE is a "self-molecule", it is generally not immunogenic. However, active immunization against IgE may be achieved by using the paratope-specific antibodies of this invention. The paratope-specific antibody shares conformational resemblance with the antigen - the ige.bl epitope - which can elicit immune response in humans against the epitope.

Paratope-specific, anti-idiotyptic antibodies are administered to a patient suffering from IgE-mediated allergy in an immunogenic amount to induce the formation of ige.bl antibodies. The anti-idiotypic antibodies are preferably administered as chimeric antibodies. They may also be given as antibody fragments (which also may be chimeric in nature) or analogues.

### 7. Peptides Which Block FcεR of Mast Cells and Basophils.

This invention also provides peptides embody the ige.bl epitopes on soluble IgE. The peptides comprise amino acid sequences which are identical or equivalent to the amino acid sequence of an ige.bl epitope of soluble IgE. For example, the peptides can have sequences corresponding to the epitopes defined by antibodies E8-5-3, E11-4-70, E101-1 and E10-12-55 or other anti-ige.bl antibodies described below. Peptides which correspond to the binding site of the FcεR can be used to block the binding of IgE to mast cells and basophils.

### 8. ε.mb/ec Peptide Analogues and Active Immunization Against ε.mb/ec Epitope

Even though human ε.mb/ec peptide is probably not immunogenic in humans, peptides with the same sequence and amino acid substitutions can be immunogenic and induce antibodies that cross react with authentic ε.mb/ec epitope. These ε.mb/ec peptide analogues can be administered to patients suffering IgE-mediated allergies. The antibodies induced by this active immunization can achieve the functions as the antibodies described herein.

9. Anchoring Peptidic piece of B Cell Membrane-bound Immunoglobulins

B cells express on their surface antibody molecules, which serve as receptors for antigens during immunological induction. The membrane-bound immunoglobulins differ from the secretory immunoglobulins synthesized by the same cells in that they have extra peptidic pieces that anchor the immunoglobulin molecules onto the cell surface.

The amino acid sequence data of the ten membrane-bound immunoglobulins from several species have been determined. See Ishida, N. et al., EMBO J., 1:1117 (1982); Steen, M. L. et al., J. Mol. Biol., 177:19-32 (1984); Rogers, J. et al., Cell, 26:19-27 (1981); Yamawaki-Kataoka, Y. et al., Proc. Natl. Acad. Sci., USA, 79:2008-2012 (1982); Kamaromy, M. et al., Nucl. Acids Res., 11:6775-6785 (1983); Rogers, J. et al., Cell, 20:303-312 (1980); Bernstein, K. E., J. Immunol. 132:490-495 (1984); Cheng, H. et al., Nature, 296:410-415 (1982). These sequences indicate certain common features of the plasma membrane bound peptidic piece. As shown in Table 1, the peptidic anchor piece has three segments which are distinguishable based upon their locations in relation to the plasma membrane. Even though these peptidic pieces are short, ranging from 41 to 72 amino acid residues, and have often been referred to as the "membrane-bound domain", the peptides are not entirely in the membrane lipid bilayer. In fact, only 25 amino acid residues, largely hydrophobic residues and threonine and serine residues, located in the middle part of the peptides, are in the lipid bilayer. The C-terminal, hydrophilic segments of 3 to 28 amino acid residues are located on the cytoplasmic side of the membrane. The segments toward the N-terminus, which are connected to the third or fourth constant domains of the immunoglobulin heavy chains ($CH_3$ or $CH_4$) are very hydrophilic and are on the extracellular side of the plasma membrane.

Table 1

| Key features and properties of peptidic segments unique to membrane-bound immunoglobulins on B cells. | | | | |
|---|---|---|---|---|
| | First segment | Middle segment | Last segment | Total |
| Immunoglobulin | Length | | | |
| Class/Subclass | # Amino acid residues | | | |
| Mouse IgE | 19 | 25 | 28 | 72 |
| Rat IgE | 19 | 25 | 28 | 72 |
| Mouse IgG$_1$ | 18 | 25 | 28 | 71 |
| Mouse IgG$_{2a}$ | 18 | 25 | 28 | 71 |
| Mouse IgG$_{2b}$ | 18 | 25 | 28 | 71 |
| Mouse IgG$_3$ | 18 | 25 | 28 | 71 |
| Mouse IgM | 13 | 25 | 3 | 41 |
| Rabbit IgM | 13 | 25 | 3 | 41 |
| Human IgD | 27 | 25 | 3 | 55 |
| Mouse IgD | 26 | 25 | 3 | 54 |
| Properties | Hydrophilic Highly acidic | Hydrophobic No charged residues | Hydrophilic | |
| Physical Location | On exterior surface | In membrane lipid bilayer | On cytoplasmic surface | |
| Abbreviated Symbols | mb/ec segment | mb/tm segment | mb/ic segment | |

\* mb for membrane-bound; ec for extracellular; tm for transmembrane; and ic for intracellular.

The shortest length of the extracellular segment of the membrane-bound pieces of the immunoglobulins (designated mb/ec segments) has 13 amino acid residues (Mouse and rabbit μ chains). See Table 2. The mb/ec segments of all immunoglobulins contain high proportions of charged amino acid residues, almost entirely acidic residues. The proportions of charged amino acid residues and polar hydrophilic residues account for very high percentages of the amino acid composition of the mb/ec segment (Table 3). These parameters indicate that all the mb/ec segments are exposed and long enough to be accessible by antibodies. Studies of the evolution of immunoglobulin heavy chains indicate the $\epsilon$ and $\gamma$ chains are more related to each other (had more recent common ancestry) than to other chains (Lien-Ching Lin and Frank W. Putnam, Proc. Natl. Acad. Sci. USA, Vol. 78 (1981), pp. 504-508). In addition, the heavy chains evolved before the various mammals species, mice, rats, and humans evolved. Thus, among the ten various mb/ec segments that have been determined, it is probably the murine or the rat $\epsilon$.mb/ec that the human $\epsilon$.mb/ec, which sequence has not yet been reported, will be most related. The next most related will be the $\gamma$ chains. The murine or rat $\epsilon$.mb/ec has 19 amino acid residues, among them 8 Glu and 2 Asp residues. These data also provide support that human $\epsilon$.mb/ec segment is exposed and accessible to antibodies.

Table 2. The amino acid sequences of the exterior portion of peptidic segments unique to membrane-bound immunoglobulins (mb/ec segments).

Mb/ec segment

| | 26 | 21 | 16 | 11 | 6 | 1 |
|---|---|---|---|---|---|---|
| Mouse IgE | | | ELDI. | QDLCI. | EEVEG. | EELEE |
| Rat IgE | | | ELDI. | QDLCT. | EEVEG. | EELEE |
| Mouse IgG$_1$ | | | GLQ. | LDETC. | AEAQD. | GELDG |
| Mouse IgG$_{2a}$ | | | GLD. | LDDVC. | AEAQD. | GELDG |
| Mouse IgG$_{2b}$ | | | GLD. | LDDIC. | AEAKD. | GELDG |
| Mouse IgG$_3$ | | | ELE. | LNGTC. | AEAQD. | GELDG |
| Mouse IgM | | | | EGE. | VNAEE. | EGFEN |
| Rabbit IgM | | | | EGE. | VNAEE. | EGFEN |
| Human IgD | YL. | AMTPL. | IPQSK. | DENSD. | DYTTF. | DDVGS |
| Mouse IgD | | I. | VNTIQ. | HSCIM. | DEQSD. | SYMDL.EEENG |

Table 3

| | TOTAL | Acidic residues | Basic residues | Polar residues | Total hydrophilic residues | Proportion of hydrophilic residues |
|---|---|---|---|---|---|---|
| Composition of charged amino acid residues and polar, hydrophilic amino acid residues in the exterior portion of peptidic segments unique to membrane-bound immunoglobulins ( mb/ec segments). | | | | | | |
| | # Amino acid residues | | | | | % |
| Mouse IgE | 19 | 10 | 0 | 2 | 12 | 63 |
| Rat IgE | 19 | 10 | 0 | 2 | 12 | 63 |
| Mouse IgG$_1$ | 18 | 6 | 0 | 4 | 10 | 56 |
| Mouse IgG$_{2a}$ | 18 | 7 | 0 | 2 | 9 | 50 |
| Mouse IgG$_{2b}$ | 18 | 7 | 1 | 1 | 9 | 50 |
| Mouse IgG$_3$ | 18 | 6 | 0 | 4 | 10 | 56 |
| Mouse IgM | 13 | 6 | 0 | 2 | 8 | 61 |
| Rabbit IgM | 13 | 6 | 0 | 1 | 7 | 54 |
| Human IgD | 27 | 6 | 1 | 8 | 15 | 56 |
| Mouse IgD | 26 | 7 | 0.5 | 9 | 16.5 | 63 |
| Acidic residues: E (Glu), D (Asp) Basic residues: K (Lys), R (Arg), H (His); His is partially charged. Polar residues: S (Ser), T (Thr), C (Cys), Q (Gln), N (Asn) | | | | | | |

10. Determining the amino acid sequence of mb/ec segment of human IgE ($\epsilon$.mb/ec segment).

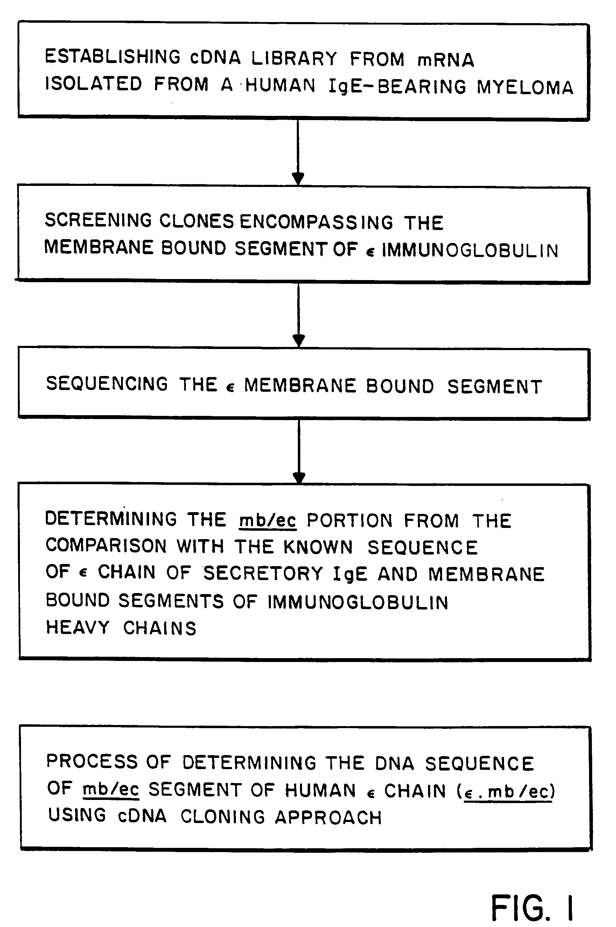

A number of well established procedures can be applied to determine the DNA sequence corresponding to the human $\epsilon$.mb/ec segment. In one approach (Figure 1), the starting point is the mRNA preparation of a human myeloma cell line which express IgE on the surface. SK007 cells can be employed for this purpose. With the mRNA preparation, one can establish a cDNA library employing cloning vector based on λ phage or plasmids. A preferred method for constructing the cDNA library is with the cDNA Library Construction System Kit - Librarian I developed and commercialized by Invitrogen (San Diego, CA). Stepwise detailed instruction manual is provided for RNA isolation from cells, reverse transcription, second strand synthesis, linker ligation, agarose gel sizing of cDNA, electroelution to purify cDNA, vector ligation, and transformation of E. coli. The vector used in this library is pCDM8.

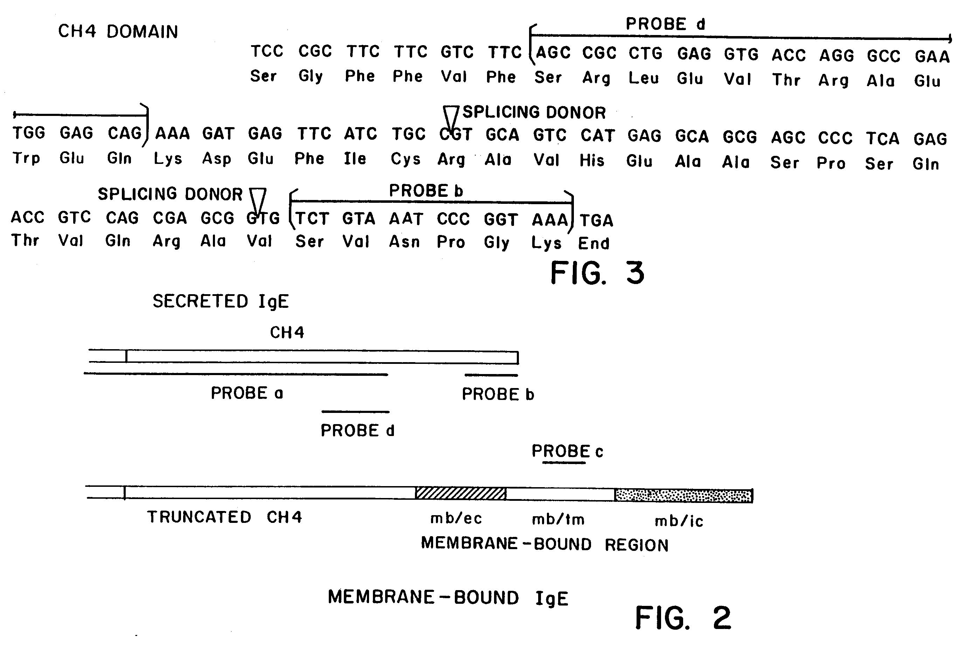

In the screening of the cDNA library for clones containing the $\epsilon$.mb/ec segment, several probes can be used. As shown in Figure 2, the library can be screened with DNA probe a, which is a 1.1 kb long U266 cDNA covering most of length of $\epsilon$ mRNA (no membrane-bound segment). The positive clones, which include both secreted and membrane-bound forms can be distinguished by using additional probes. Probe b is developed by taking advantage of the very likely property that the end of the CH$_4$ domain is truncated in the human $\epsilon$ chain of the membrane-bound form. The truncation occurs when the gene segments of CH$_4$ domain and membrane bound domain are translocated. The loss of C-termini occurs with the membrane bound forms of immunoglobulins, including $\epsilon$ and $\mu$, which contain CH$_4$ domains. From the published information on nucleotide sequence of human $\epsilon$.CH$_4$ domain, the most possible splicing donor site is intracodon GT, 71 bp 5' of the termination codon TGA. Another GT, which is not intracodon and less likely a splicing donor site, is closer to the terminus (24 bp 5' to the termination codon).

The specific location for probe b is indicated in Figure 2 and Figure 3. The probe will react with secreted form of $\epsilon$ chain gene and not membrane form of $\epsilon$ chain gene.

The design of probe c is based on the finding that the transmembrane segment of the membrane-bound domain (mb/tm segment) is very conserved among all the immunoglobulin genes so far sequenced. There is a segment of peptide and its corresponding coding DNA within this mb/tm segment, that is nearly identical among all immunoglobulins. As shown in Table 4, the consensus DNA sequence with the eight combinations will be used as probe c. Figure 2 shows the location of the probe.

9

Table 4.  A conserved region in the transmembrane
portion of the peptidic segment of the
membrane-bound immunoglobulins (in mb/tm
segment).

|  | 1 | 2 | 3 | 4 | 5 |
|---|---|---|---|---|---|
|  | Leu. | Phe. | Leu. | Leu. | Ser. |
| Mouse IgE | CTG. | TTC. | CTG. | CTC. | AG |
| Rat IgE | CTG. | TTC. | CRG. | CTC. | AG |
| Mouse $IgG_1$ | CTC. | TTC. | CTG. | CTC. | AG |
| Mouse $IgG_{2a}$ | CTC. | TTC. | CTG. | CTC. | AG |
| Mouse $IgG_{2b}$ | CTC. | TTC. | CTG. | CTC. | AG |
| Mouse $IgG_3$ | CTC. | TTC. | CTG. | CTC. | AG |
| Mouse IgM | CTC. | TTC. | CTC. | CTG. | AG |
| Rabbit IgM | CTG. | TTC. | CTG. | CTG. | AG |
| Human IgD* | CTC. | TTC. | ATC. | CTC. | AC |
| Mouse IgD* | CTC. | TTC. | CTG. | CTC. | AC |

```
                      C           G     C
Consensus         CT .TTC.CT .CT .AG
   sequence        G           C     G
  (Probe c)
```

\*Human and mouse IgD's have Thr (ACX0) in the
5th amino acid residue; human IgD also has Ile
(ATC) in the 3rd amino acid residue.  These are
the variations not covered by the consensus
sequence.

Probe d which represents a segment up-
stream to the most possible splicing donor

site, GT, is consisted of 36 bp (Figure 2 and
Figure 3).  This probe should react with $\epsilon$
chain gene of both secreted and membrane-bound
forms.

Table 5 summarizes the pattern of reactivities of clones containing $\epsilon$ genes of secreted or membrane-bound forms with the four probes.

Table 5

| The reactivity of $\epsilon$ gene-containing cDNA clones with probes a, b, c, and d. | | |
|---|---|---|
| | $\epsilon$ Secreted | $\epsilon$ Membrane-bound |
| Probe a | + | + |
| Probe b | + | - |
| Probe c | - | + |
| Probe d | + | + |

The library size needed to clone the membrane-bound $\epsilon$ chain depends on how abundant the mRNA is. Assuming secreted IgE comprises 0.1% of the SK007 poly $A^+$ RNA, the library size should be about 5,000 independent recombinant clones to have a 99% possibility to isolate a positive clone. In IgE-producing rat immunocytoma IR2 and IR162 cells, mRNA for the membrane-bound form of $\epsilon$ chain was found to be more than 2% of that of the secreted form. Assuming this ratio of membrane-bound/secreted forms of $\epsilon$ chain holds true for the human IgE-producing SK007 cells, the cDNA library size needed to isolate the membrane-bound $\epsilon$ chain is about 250,000. In a preferred procedure, a larger number of clones, 1,000,000, are screened.

An alternative to the conventional approach of establishing cDNA library and screening the clones representing the cellular mRNA species is to amplify the mRNA by producing high proportions of their corresponding DNA. The resulting DNA can then be purified by gel electrophoresis and then subjected to sequence analysis. The methodology, referred to as polymerase chain reaction (PCR) amplification, has been established in the past few years and complete systems including reagents and equipments have been commercialized. One preferred system is provided by Perkin Elmer Cetus (Norwalk, CT). The reagents kit is the GeneAmp DNA Amplification Reagent Kit and the equipment is the DNA Thermal Cycler.

Some of the specific reagents used in this approach are the same as used for the cDNA library cloning. Since no sequence in the membrane-bound segment of the $\epsilon$ chain has been determined, the strategy is to amplify both the secreted and membrane-bound forms of $\epsilon$ chains. Two primers are to be used, one is oligo-dT (25-30-mers) and one is the oligomer corresponding to probe d in Figure 2 and Figure 3. Probe d is located 5' to the most possible splicing donor site and therefore primes both the secreted and membrane-bound forms of $\epsilon$ mRNA and DNA. After sufficient amplification, the two populations of DNA fragments are resolved by gel electrophoresis. The secreted form of the e chain can be distinguished by its reactivity with probe b. The purified DNA's are then subjected to DNA sequencing.

PCR amplification seems to be a more efficient procedure than cDNA cloning for mRNA poorly represented in the poly $A^+$ RNA pool. The U266 $\epsilon$ chain cDNA can be used to work out some preliminary annealing conditions between template DNA and oligo-primers.

Another approach of obtaining a DNA clone containing genes encoding the membrane-bound segments is to screen human genomic DNA library. Human genomic DNA library is readily available. A preferred source is the library constructed using human lung fibroblast WI38 cells provided by Stratagene (La Jolla, CA). The genes are in $\lambda$ vector and the inserted DNA have average sizes of 15K bp. Identification of the clones can be achieved by hybridization with U266 cDNA clone DNA. The location of the gene segment corresponding to the membrane bound region can be determined by using probe prepared from the homologous mouse gene of the transmembrane segment (probe c of Figure 2 and Table 4). The sequence of the membrane-bound segment is then determined.

10A. The nucleotide sequence of DNA encoding membrane anchoring peptide of human $\epsilon$ chain

Nucleotide sequence of genomic DNA encompassing the encoding segments for the membrane anchoring peptide of human membrane bound $\epsilon$ chain was determined and is shown below along with the deduced amino acid sequence for portions of the membrane anchoring peptide. The assignment of the exons was made by identifying the nucleotides for splicing donors and acceptors and by comparing to the published homologous sequences of mouse membrane bound $\epsilon$ chain and of immunoglobulins of other classes.

```
·····CGCTGCCACTGTGGAGCCGGGAGGGCCTGACTGGCCAG

        splice-acceptor
GTCCCCCAGAG·CTG·GAC·GTG·TGC·GTG·GAG·GAG·GCC·GAG·
        Glu·Leu·Asp·Val·Cys·Val·Glu·Glu·Ala·Glu·

GGC·GAG·GCG·CCG·TGG·ACG·TGG·ACC·GGC·CTC·TGC·ATC·              Membrane
Gly·Glu·Ala·Pro·Trp·Thr·Trp·Thr·Gly·Leu·Cys·Ile·            exon I

TTC·GCC·GCA·CTC·TTC·CTG·CTC·AGC·GTG·AGC·TAC·AGC·
Phe·Ala·Ala·Leu·Phe·Leu·Leu·Ser·Val·Ser·Tyr·Ser·

                        splice-donor
GCC·GCC·CTC·ACG·CTC·CTC·ATG·GTGGGCACCCACCTCCAGG
Ala·Ala·Leu·Thr·Leu·Leu·Met·

GGCCCAGCCAGGGCAGGGGGTTGGGCAGAGCAGCAGAGCCCCTGACC              Intron

        splice-acceptor
CACGCCCTCCCCTCAGGTG·CAG·CGG·TTC·CTC·TCA·GCC·ACG·
                Val·Gln·Arg·Phe·Leu·Ser·Ala·Thr·

CGG·CAG·GGG·AGG·CCC·CAG·ACC·TCC·CTC·GAC·TAC·ACC·              Membrane
Arg·Gln·Gly·Arg·Pro·Gln·Thr·Ser·Leu·Asp·Tyr·Thr·            exon II

AAC·GTC·CTC·CAG·CCC·CAC·GCC·TAG·GCCGCGGGCACTCAC
Asn·Val·Leu·Gln·Pro·His·Ala·end

GCTCCACCAGGCCCAGCTACCC·····
```

Human ε.mb/ec peptide is identified as the first fourteen amino acids encoded by membrane exon I. This precedes a stretch of about 25 hydrophobic amino acids which are the transmembrane region. Two possibles structures of ε.mb/ec are shown below.

Possible Structures of Human ε.mb/ec peptide

**Structure I**

```
                                SH
·····Glu·Leu·Asp·Val·Cys·Val·Glu·Glu·Ala·Glu·Gly·Glu·Ala·Pro·····
```

**Structure II**

```
·····Glu·Leu·Asp·Val·Cys·Val·Glu·Glu·Ala·Glu·Gly·Glu·Ala·Pro·····
                      S
                      S
·····Glu·Leu·Asp·Val·Cys·Val·Glu·Glu·Ala·Glu·Gly·Glu·Ala·Pro·····
```

As described more fully below, the ε.mb/ec peptide can be used to elicit antibodies which react specifically with membrane bound immunoglobulin E. For this purpose, the peptides can be chemically synthesized by standard techniques of protein synthesis. A preferred method for synthesizing the peptides is with RaMP system (DuPont, Wilmington, DE), which applies Fmoc chemistry. Alternatively, the proteins can be biosynthesized by employing oligodeoxynucleotides encoding the peptide. The nucleotide sequence is given as the 5' portion of membrane exon I, above.

As immunogens, the proteins may be used in either the monomeric or dimeric structural forms shown above. Peptides comprising the human ε.mb/ec segment and the connecting four amino acids in the CH4 domain can also be used. In addition, modified peptides having subtantial immunological equivalency can be used. For example, the peptide amino acid sequence shown above can be modified by deletion, insertion or substitution of one or more amino acids which do not essentially detract from the immunological properties of the peptide. The peptides can also be used as polymers where the amino acid sequence shown above, or equivalent sequence, is the polymer repeat unit.

11. Production of the Monoclonal Antibodies of this Invention.

The monoclonal anti-IgE and anti-idiotypic antibodies of this invention are produced by continuous (immortalized), stable, antibody-producing cell lines. The preferred antibody-producing cell lines are hybridoma cell lines and myeloma cell lines. In principle, however, the cell lines can be any cells which contain and are capable of expressing functionally rearranged genes which encode the antibody variable regions of the antibody light and heavy chains. Preferably, the cell line should have the capability to assemble the chains into functional antibodies or antibody fragments. Thus, lymphoid cells which naturally produce immunoglobulin are most often employed. In addition to those previously mentioned, examples of suitable lymphoid cells are viral or oncogenically transformed lymphoid cells.

Hybridoma cells which produce the ige.bl-specific antibodies of this invention can be made by the standard somatic cell hybridization technique of Köhler and Milstein, Nature 256:495 (1975) or similar procedures employing different fusing agents. Briefly, the procedure is as follows: the monoclonal anti-IgE antibodies are produced by immunizing an animal with human IgE, or peptidic segments of human IgE (secretory or membrane-bound), which are identified as potential components of ige.bl epitope. The peptides can be synthesized and conjugated to a carrier protein, such as keyhole limpet hemocyanin, to be used as an immunogen. Lymphoid cells (e.g. splenic lymphocytes) are then obtained from the immunized animal and fused with immortalizing cells (e.g. myeloma or heteromyeloma) to produce hybrid cells. The hybrid cells are screened to identify those which produce the desired anti-IgE antibody.

Human hybridomas which secrete human antibody can be produced by the Köhler and Milstein technique. Although human antibodies are especially preferred for treatment of humans, in general, the generation of stable human-human hybridomas for long-term production of human monoclonal antibody can be difficult. Hybridoma production in rodents, especially mouse, is a very well established procedure. Stable murine hybridomas provide an unlimited source of antibody of select characteristics. Murine antibodies, however, may have limited use in the treatment of humans because they are highly immunogenic and can themselves induce untoward allergic reactions in the recipient. In the preferred embodiment of this invention, the anti-IgE and anti-idiotypic antibodies are produced in a rodent system and are converted into chimeric rodent/human antibodies by the established techniques described in detail below. As explained above, these "near human", chimeric antibodies are preferred for in vivo administration, especially where multiple doses are required.

For the production of the antibodies specific for ige.bl eptiopes on soluble IgE human IgE for immunization can be purified from human serum. Alternatively, human IgE may be produced by culturing an IgE-producing cell line (for example, the cell line U266, ATCC number CRL8033). Human IgE can be purified by affinity chromatography. Mouse monoclonal antibodies specific for human IgE are conjugated to a suitable matrix (such as cyanogen bromide-activated Sepharose 4B) to provide an IgE-specific immunoadsorbent. The IgE preparation can be contacted with the immuno- adsorbent which selectively adsorbs IgE. The adsorbed IgE can thereafter be eluted in substantially pure form from the immunoadsorbent.

In preferred embodiments, animals are immunized with a vigorous immunization protocol in order to produce a high frequency of lymphocytes producing IgE-specific antibodies. Spleen cells are obtained from the immunized animal and fused with an immortalizing cells, preferably myeloma cells which have lost the ability to secrete immunoglobulin. Many suitable myeloma cell lines are known in the art. An example is the murine myeloma NS-1. Fusion of the spleen cells and fusion partner can be carried out in the presence of polyethylene glycol according to established methods. Techniques of electrofusion may also be used. The resulting hybrid cells are clonally cultured and then screened for production of anti-IgE antibody.

Hybridomas producing antibodies which are specific for an epitope present on IgE-bearing B cells and absent on basophils and which have an affinity for IgE sufficient to block FcεR binding to IgE can be selected as follows. Hybridomas are first screened for production of antibody reactive with human IgE. This can be done by an enzyme-linked immunosorbent assay (ELISA) employing purified human IgE adsorbed to a solid phase.

13

One way of obtaining generally high affinity antibodies is as follows. The solid phase for the ELISA is coated with very small amounts of human IgE. For example, if a standard microwell plate is used as the solid phase, about 50 $\mu$l of a 0.1 $\mu$g/ml solution of IgE is used per well. Hybrids are selected which show a comparatively high enzyme activity (optical density level) in the assay. The culture supernatants contain either relatively higher amounts of antibodies or antibodies of relatively higher affinity or both.

Hybridomas are then screened for secretion of antibodies which do not react with basophil-bound IgE. A preferred method is to screen the antibodies for the inability to induce histamine release by basophils. The source of basophils for such histamine release assays is blood leukocytes from donors whose basophils are known to be very sensitive for induction of histamine release. An alternative and possibly less sensitive method is an immunofluorescence staining technique. Basophil leukocytes can be isolated from blood. Freshly isolated basophils have IgE on their surface. Monoclonal antibodies which do not bind basophil-bound IgE are specific for an epitope which is at or near a site occupied by the basophil Fc$\epsilon$R (and hence is inaccessible for the monoclonal antibodies).

Hybridomas which produce paratope-specific anti-idiotypic antibody can be made by immunizing an animal with anti-IgE antibody and screening for antibodies which bind the paratope of the immunizing anti-IgE antibody. Immunization results in production of antibodies against the antigenic determinants on the anti-IgE antibody including the idiotype. Anti-idiotype antibodies are first screened for their binding to anti-IgE antibody and not other mouse antibodies. Those which are paratope-specific are screened on the basis of the antibody's ability to compete the binding of human IgE to the anti-IgE monoclonal antibody used for immunization.

Antibody fragments such as F(ab')$_2$, Fab and F$_v$ can be produced by standard techniques of enzyme digestion. In addition, synthetic peptides representing Fab and F$_v$ analogues can be produced by genetic engineering techniques. See e.g., Better, M. et al. (1988) Science 240:1041; Huston, J.S. et al., (1988) Proc. Natl. Acad. Sci. USA 85:5879-5883.

The chimeric anti-IgE antibodies are comprised of individual chimeric heavy and light immunoglobulin chains. The chimeric heavy chain is a contiguous polypeptide having a rodent (generally murine) heavy chain variable region or hypervariable regions and a human heavy chain constant region. The chimeric light chain is a contiguous polypeptide having a rodent light chain variable regions or hypervariable regions and human light chain constant region.

The chimeric antibodies can be monovalent, divalent or polyvalent. Monovalent antibodies are dimers (HL,) formed of a chimeric heavy chain associated (through disulfide bridges) with a chimeric light chain. Divalent immunoglobulins are tetramers (H$_2$L$_2$) formed of two associated dimers. Polyvalent antibodies can be produced, for example, by employing heavy chain constant region which aggregate (e.g., mu type constant regions).

The variable regions of the chimeric antibodies are derived from the anti-IgE antibody of this invention. The heavy chain constant region can be selected from any of the five isotypes alpha, delta, epsilon, gamma or mu. Heavy chains of various subclasses (such as the IgG subclasses) can be used. The different classes and subclasses of heavy chains are responsible for different effector functions and thus, by choosing the desired heavy chain constant region, chimeric antibodies with desired effector function can be produced. The light chain constant region can be from the kappa or lambda chain.

In general, the chimeric antibodies are produced by preparing a DNA construct which encodes each of the light and heavy chains components of the chimeric antibody. The construct comprises fused gene comprising a first DNA segment which encodes at least the functional portion of the murine variable region (e.g. functionally rearranged variable regions with joining segment) linked to a second DNA segment encoding at least a part of a human constant region. Each fused gene is assembled in or inserted into an expression vector. Recipient cells capable of expressing the gene products are then transfected with the genes. The transfected recipient cells are cultured and the expressed antibodies are recovered.

Genes encoding the variable region of rodent light and heavy chains can be obtained from the hybridoma cells which produce the anti-IgE antibodies. For example, the murine hybridoma cell lines which produce murine anti-IgE antibody provide a source of variable region genes.

Constant regions genes can be obtained from human antibody producing cells by standard cloning techniques. Alternatively, because genes representing the two classes of light chains and the five classes of heavy chains have been cloned, constant regions of human origin are readily available from these clones.

Preferably, the fused genes encoding the light and heavy chimeric chains are assembled into expression vectors which can be used to cotransfect a recipient cell. Suitable vectors for the gene constructs include plasmids of the types pBR322, pEMBL and pUC. Each vector contains two selectable genes - one for selection in a bacterial system and one for selection in a eukaryotic system - each vector having a different pair of genes. These vectors allow production and amplification of the fused genes in bacterial systems and subsequent cotransfection of eukaryotic cells and selection of the cotransfected cells. Examples of selectable gene for the bacterial system are the genes which confer ampicillin and the gene which couples chloramphenicol resistance. Examples of selectable genes for eukaryotes are gpt and neo.

The preferred recipient cell line is a myeloma cell. Myeloma cells transfected by antibody genes can synthesize, assemble and secrete immunoglobulins encoded by the antibody genes.

Further, they possess the mechanism for glycosylation of the immunoglobulin. A particularly preferred recipient cell is the Ig-nonproducing myeloma cell SP2/0. Shulman et al, Nature 276:269 (1978). The cell produces only immunoglobulin encoded by the transfected immunoglobulin genes. Myeloma cells can be grown in culture or in the peritoneum of mice where secreted immunoglobulin can be obtained from ascites fluid. Other lymphoid cells such as B lymphocytes or hybridoma cells can serve as suitable recipient cells.

Lymphoid cells can be transfected with vectors containing immunoglobulin encoding genes in several ways. These include electroporation, protoplast fusion and calcium phosphate precipitation procedure. The resulting transfected cells provide continuous, stable cell lines which produce chimeric antibodies.

The chimeric antibodies can be produced in large quantity in large scale tissue culture systems such as various continuous perfusion systems, hollow fiber systems, static maintenance culture systems or other systems.

Near human antibodies or antibody fragments can also be produced by engineering gene sequences of human antibodies which encode the hypervariable (complementarity determining) regions providing appropriate anti-IgE specificity. See e.g., Robert, S. et al., Nature 328:731-733 (1987); Better, M. et al., (1988) Science 240:1041.

12. Developing Antibodies to mb/ec Segment.

The ε.mb/ec peptides can be used in the immunization of animals to prepare polyclonal and monoclonal antibodies. They can also be used to screen for specific monoclonal antibodies or characterize specific polyclonal antibodies. They can also be used to purify monoclonal and polyclonal antibodies.

In the process of preparing for monoclonal antibodies specific for ε.mb/ec peptide, it is not necessary to use the ε.mb/ec peptide in both immunization and antibody identification. For example, in immunizing mice for preparing immune spleen cells for fusion with myeloma cells, the immunogen may be the membrane-bound IgE isolated from plasma membrane of IgE-bearing myeloma cells, such as SK007 cells. The immunogen may also be the IgE-bearing myeloma cells.

For using the synthetic ε.mb/ec peptide for immunogen, it is more effective to conjugate the peptide to a protein carrier. A preferred protein carrier is keyhole limpet hemocyanin (KLH). If the peptidic segment lacks a Lys residue or if the Lys residue is in the middle part of the segment, it is desirable to add a Lys residue at the C-terminal end. Because the N-terminus already has an α-amino group, the modified synthetic peptide will have two amino groups for linking.

Multiple molecules of peptides can be conjugated to each molecule of the carrier protein. With KLH, a preferred molar ratio for peptide/KLH is 10. The method of conjugation is very well established. Cross-linkers such as glutaldehyde or bis (sulfosuccinimidyl) suberate or disulfosuccinimidyl tartarate (Catalogue #21579, 20591, Pierce Chemical Co., Rockford, IL) have been used. A preferred cross-linker is the latter.

The immunogen, such as the KLH conjugate, can be used to immunize rabbits, goats, rats, or mice to prepare polyclonal antibodies specific for the ε.mb/ec peptide. Lymphocytes from the spleen or lymph nodes of immunized mice and rats can also be taken to prepare hybridomas secreting monoclonal antibodies specific for the ε.mb/ec peptide. A preferred protocol to prepare the monoclonal antibodies is to fuse immune spleen cells of mice with non-secreting mouse myeloma cells, such as NS-1 or SP2/0 cells using polyethylene glycol.

For optimal immunization of mice, 50 μg of the peptide-KLH conjugate in complete Freund's adjuvant is injected subcutaneously into each mouse for priming. Two and four weeks later, the same amount of antigen is given s.c. in incomplete Freund's adjuvant. At about the six week time point, the fourth antigen injection is given i.p. in saline. Mice are sacrificed 4 days after the last injection and the spleens are taken for preparing single cell suspension for fusion with myeloma cells. Similar protocols can also be used for immunization with purified native human membrane-bound IgE (having attached membrane anchor domain) isolated from the plasma membrane of IgE-bearing human myeloma cells, such as SK007 cells. When human IgE-bearing cells are used as the immunogen, 1x10$^7$ cells are injected i.p. with two week intervals.

The fusion procedure with polyethylene glycol and other various procedures concerning cloning and hybridoma culturing have been described in Section 11.

The screening of hybridomas for monoclonal antibodies or the identification of polyclonal antibodies reactive with ε.mb/ec peptide can be performed with enzyme linked immunosorbent assays (ELISA) using the synthetic ε.mb/ec peptide as the solid phase antigen. An alternative solid phase antigen is the conjugate of ε.mb/ec peptide with a different carrier protein such as bovine serum albumin different from that used in immunogen. Further characterization of the monoclonal and polyclonal antibodies are shown in Table 6. The assays employed in these studies are also indicated. The assays are described below.

Table 6

| The reactivity of antibodies specific for ε.mb/ec peptide with different IgE-containing targets. | | |
|---|---|---|
| | Reactivity | Assays |
| Synthetic ε.mb/ec peptide | + | ELISA |
| Soluble IgE | - | ELISA |
| Basophils and mast cells | - | Immunofluorescence staining Histamine release |
| SK007 myeloma cells | + | Immunofluorescence staining |
| IgE-bearing B cells | + | Immunofluorescence staining |

13. Experiments with Animal Models.

The substances and methods are tested on animal model systems. Two of the most relevent systems are the following.

A. Asthma/rhesus monkey model

The monoclonal antibodies specific for human ε.mb/ec peptide and their related substances of this invention are intended for use to treat patients with various IgE-mediated allergies (see section 6 below). Among these allergies, extrinsic asthma is a more serious form. An experimental model system for studying asthma has been established with rhesus monkeys.

A small portion of rhesus monkeys, which have been infected with the nematode, Ascaris suum, develop sensitivity to extract of ascaris. When these sensitive monkeys are given spray containing ascaris antigen, they develop breathing problems resembling asthma. Patterson, R.,J. Clini. Invest. 57:586-593 (1976).

The various substance of this invention can be experimented in the asthma/rhesus monkey model system. The ascaris sensitive monkeys are given the experimental treatment or control treatment and measurements are made to determine:

(a) Does the asthma symptoms upon ascaris challenge decline?
(b) Does the circulating IgE decline?
(c) Does the circulating IgE-bearing B cells decline?
(d) Does the IgE density on basophils decline?

B. Mouse model system

Mice are not known to develop allergic symptoms naturally. However, for demonstrating the pharmacologic mechanisms of the intended therapy in regards to the depletion of IgE-bearing B cells and IgE, the mouse can serve as an excellent model.

The ε.mb/ec segment of mouse has already been sequenced. Ishida, N. et al., EMBO J. 1:1117-1123 (1982) The 19 amino acid residue peptide is

```
Glu-Leu-Asp-Leu-Gln-Asp-Leu-Cys-Ile-Glu-Glu-Val-

Glu-Gly-Glu-Glu-Leu-Glu-Glu
```

The peptide is synthesized in several forms, including one that has extra Leu-Lys residues at the C-terminus.

The peptide and its KLH conjugate are used as antigens to immunize rabbits and goats. The anti-sera are collected. The antigen-specific antibodies are purified using a column of Sepharose 4B conjugated with the peptide (with Leu-Lys addition) or with peptide linked to bovine serum albumin. Normal mice are injected i.v. or i.p. with the purified antibodies or their related substances to study the following questions:

(a) Does the total IgE in circulation decline?

(b) Does the number of IgE-bearing B cells decline?

(c) Does the density of IgE on the surface of basophils decline?

(d) Do IgM and IgG specific for $\epsilon$.mb/ec peptide cause different effects? The purpose of this question is to address the effects of antibody-dependent cellular cytotoxicity (ADCC) in the depletion of IgE-bearing B cells. IgG, not IgM is known to mediate ADCC.

The invention is further illustrated by the following examples.

Example I: Preparation of the Hybridomas and Monoclonal Antibodies

a) Preparation of Human IgE

Human IgE was obtained from a commercial source and purified for immunizing mice to obtain immune splenocytes for fusion and for screening hybrids. The IgE was also used to characterize the various monoclonal anti-IgE antibodies. Two preparations of human IgE were used. One was polyclonal IgE purified from human sera, which was obtained from Ventrex (Portland, Maine). This human IgE was purified from sera by affinity chromatography using Sepharose 4B column conjugated with rabbit IgG specific for human IgE. Contaminating human albumin and transferrin were removed by affinity column conjugated with antibodies specific for albumin and transferrin. Monoclonal human IgE was also produced from culture supernatants of IgE-producing U266 cell line. The IgE was affinity purified on a Sepharose 4B column conjugated with a monoclonal antibody specific for human IgE. This monoclonal antibody IgG was purified from the ascitic fluids of mice bearing the specific hybridomas with a protein A-conjugated column .

The polyclonal and monoclonal human IgE's were analyzed by SDS-polyacrylamide gel electrophoresis under reducing and non-reducing conditions. In both cases, distinctive IgE molecules (under non-reducing conditions) and heavy and light chains (under reducing conditions) were observed and only traces of relatively very light bands of some other contaminating proteins were present. Since the U266 cell line was grown in serum-free, defined medium, we had some clues as to the identity of the contaminating proteins in the monoclonal human IgE preparation.

b. Immunization Procedure

1. Procedure

Male Balb/c mice of initially 6-8 weeks old were used for immunization for preparing immune spleen cells for fusion with myeloma cells to produce hybrids. The polyclonal human IgE purified from sera provided by Ventrex was used as the immunogen. The rationale for this is that the monoclonal IgE produced by U266 cell line might bear certain unknown anomalies. In addition, we did not want to generate monoclonal antibodies against the idiotypes of U266 IgE, and it would be much more likely to induce anti-idiotypic responses against monoclonal antibodies. After performing three fusion experiments with mice immunized with U266-derived IgE, we switched to fusions with mice immunized with polyclonal, human-sera-derived IgE.

For immunization, each mouse was injected with 50 $\mu$g of human IgE per injection. The first immunization was given in complete Freund's adjuvant. The mice were injected subcutaneously at sites with high concentrations of lymph nodes, for example, the underside of the intersection of the limbs and the trunk. One month and two months later the mice received subcutaneous booster injections at the same sites with 50 $\mu$g IgE. The boosters were prepared essentially in the same manner as was the first injection, except that for the boosters the emulsification was done in incomplete Freund's adjuvant.

After at least another month, each mouse was reimmunized subcutaneously for the last time (the fourth injection) with 50 $\mu$g IgE in PBS. Each mouse was injected subcutaneously at the intersection of each limb with the trunk, and intraperitoneally. Three days after the last injection, the mice were sacrificed and their spleens were removed. The spleen cells were then fused with myeloma cells by the following procedure.

c) Fusion

Suspensions containing a five-to-one ratio of spleen cells to myeloma cells were prepared. The myeloma cells chosen were NS-1. The NS-1 cells were conditioned to have a doubling time about every seventeen hours. They were used for fusion when in the log phase. The NS-1 cells were subcultured in bacteriological plates (100mm) at a concentration of 6 x $10^4$ cells/ml in 10 ml of Dulbecco's Modified Eagle's Medium (DMEM) containing 5% fetal bovine serum (FBS), 100 units/ml of penicillin and 100 ug/ml of streptomycin. The medium was changed every three days. Alternatively, the cells were subcultured at 1.5 x $10^5$ cells/ml in 10ml of the same medium, and the medium was changed every two days.

The spleen cells were prepared by placing the spleen on a bacteriological plate (100 mm) and injecting 20 ml of calcium, magnesium free PBS (CMF-PBS) into both ends of the spleen to loosen up the spleen cells. The spleen cells were then transferred to a 50 ml centrifuge tube.

The spleen cells were centrifuged at 200 g for five minutes, and then suspended in 5 ml of 0.83% $NH_4Cl$ (0.155 M) for 10 minutes at room temperature to lyse the erythrocytes. 5 ml of CMF-PBS was added to the tube to stop the lysis. The cells were then pelleted and resuspended in 10 ml of CMF-PBS.

The concentration of lymphocytes was determined by adding 40 $\mu$l of cell suspension to 10 ml of saline together with 3 drops of Zap-oglobin™. The number of lymphocytes was counted with a hemacytometer and the concentration of cells determined.

The NS-1 cells were transferred from bacteriological plates (100 mm) to a 50 ml centrifuge tube. The cell concentration was determined. The NS-1 cells were then suspended in 10 ml of CMF-PBS and mixed with spleen cells at 1:5 in a 50 ml centrifuge tube. Routinely, we obtain 2-5 x $10^8$ cells from one spleen from an immunized animal and we use two spleens in each fusion experiment.

The cells were spun down and washed once with 10 ml of CMF-PBS. The supernatant was aspirated as much as possible with a glass Pasteur pipette. The tube was gently tapped to free the cell pellet.

Prior to preparing the cells, a fusion mixture had been prepared as follows. 5 g of polyethylene glycol 1450 (Kodak) had been mixed with 5 ml of CMF-PBS and 0.5 ml of DMSO. This mixture had been warmed to 56°C, titrated to a final pH of 7.0, and filtered through a 0.22 $\mu$ Millipore filter to sterilize. 1.0 ml aliquots had been added to Cryotubes, and these had been stored at -70°C.

To prepare the fusion mixture for use, one of the aliquots in the Cryotubes was melted by heating it to 37°C. Separately, a tube containing 10.0 ml of DMEM (without serum) was heated to 37°C.

The 1.0 ml aliquot of polyethylene glycol fusion mixture was added to the cell suspension and the suspension was mixed well. Forty-five seconds after the polyethylene glycol fusion mixture had been added, 2.0 ml of the preheated DMEM (without serum) was added dropwise with mixing. The remaining 8 ml of the pre-heated DMEM (without serum) was then added. The cells were left at room temperature for 10 minutes.

2.0 ml of FBS was added to the suspension and the suspensions were mixed well. The combination of the FBS and the CMF-PBS can help to prevent adherence of cells to the test tube walls. The suspension were then centrifuged at 400 g for four minutes.

After having been spun down, the cells were suspended in about 120 ml of a modified medium, supplemented with 5% FBS, 100 units/ml of penicillin, 100 ug/ml of streptomycin, and hypoxanthine, aminopterin and thymidine (HAT).

The concentration of the cell suspension was adjusted to 3.3 x $10^5$ of the spleen cells per 200 microliters of suspension. 200 microliter aliquots of suspension were then distributed to each well of a 96 well microtiter plate. After typically 20-30 such plates were prepared for each fusion, the plates were transferred to an incubator and maintained at 37°C in 5% $CO_2$.

The cells were grown for seven days in the plates, then the growth medium was withdrawn and new medium was added. Four days after that, an enzyme linked immunosorbent assay (ELISA, below) was performed on the antibodies in the wells to determine which would bind human IgE.

## 2. Results

Four fusion experiments with mice using the above immunization protocols were done. For these fusions, 7, 15, 36, and 15 plates of 96 wells of fusion cells were prepared, respectively. More than 98% of wells had cell growth and a well had on the average 3-5 clones of hybrids. Thus, we produced about 7,000 wells and probably 21,000-35,000 clones in the four fusions.

### d) ELISA Procedure

### 1. Procedure

The primary screening procedure for the very large numbers of hybrids resulting from the fusion wells was ELISA with human IgE as the solid phase antigen. The polyclonal IgE purified from human sera (Ventrex) was used as the antigen.

One important and decisive strategy in our screening procedure was to screen generally high affinity antibodies from the 1,000-4,000 wells from each fusion experiment. This was done by coating very small amounts of human IgE, 50 $\mu$l of 0.1 $\mu$g/ml onto each well. Assuming all the IgE was bound to the solid phase, only 5 ng would be in each well. Because of this small amount, the possibility of screening out hybrids specific for contaminating proteins was also greatly reduced. Another very important strategic point was that only wells that show high O.D. readings were chosen for further characterization and for cloning.

In the procedure, 50 $\mu$l of 0.1 $\mu$g/ml of human IgE was added to wells of 96-well Immunlon I plates. The plates were covered and incubated for eighteen hours at 4°C to allow the protein to bind to the plate.

The liquid contents of the plates were then emptied, and 200 $\mu$l of 0.1 M $NH_4Cl$ was added to each well in order to saturate any remaining binding sites on the plates. The $NH_4Cl$ solution was left in the wells for 30 minutes at room temperature.

The $NH_4Cl$ solution was then removed and the wells were washed three times with PBS and 0.05% Tween 20. Some of the PBS/0.05% Tween 20 solution was left in the wells until the antibody suspension described below was added.

50 $\mu$l of the cell fusion supernatant from each well of the 96 well plates was added to each of the wells on the Immulon I plates, and incubated for one hour. Following incubation, the plates were rinsed three times with PBS/0.05% Tween 20 in order to remove any unbound antibody.

The cell fusion supernatant would contain the antibody which was produced by the various hybridomas in the 96 well plates. The antibody which was specific to human IgE would bind thereto. The amounts of antibodies bound to the solid phase were then determined by a routine procedure using horseradish peroxidase-conjugated goat-anti-mouse IgG, using 3,3',5,5'-tetramethyl benzidine as the substrate.

2. Results

From the approximate 7,000 wells screened totally, about 4,000 wells (about 60%) were positive in the ELISA. Most of these positive wells probably contained hybrids producing monoclonal antibodies for human IgE. From these approximately 4,000 wells in the ELISA, we chose 53 wells with the highest O.D. readings for cloning and further characterization.

The 53 monoclonal antibodies were checked in ELISA using wells of plates coated with human serum at various dilutions. All of them were negative in the ELISA suggesting that they did not react with human albumin, IgE, IgM, transferrin, or other major serum proteins which might have contaminated the IgE preparations used as the immunogen for mice and as the antigen in the primary screening ELISA.

d) Single Cell Cloning

Cell suspensions from each of the 53 wells with the highest O.D. readings in ELISA were expanded in the wells of a twenty-four well plate, the cell suspensions were diluted to thirty, fifty and one hundred cells per milliliter. 0.1 ml of the diluted cell suspensions (containing an average of three, five and ten cells, respectively) was placed into the wells of a 96-well plate. The wells had been coated with histone.

After the cells grew up to become colonies, the cells were checked under a microscope. The cells of each colony did not move about and form colonies. The single-cell clones showing strongest reactivities in ELISA were chosen and expanded in culture.

e) Production and Purification of Monoclonal Antibodies

To produce large quantities of desired monoclonal antibodies, the following procedure was performed.

Some of the clones showing high O.D. readings in ELISA, which were grown in the wells in the twenty four well plates, were expanded further in 100 mm tissue culture plates. The expanded culture of the selected single-cell clones were then separately injected into the peritoneal cavity of prestane treated mice, using five million cells per mouse. After seven days the ascites fluid of each mouse was collected and frozen.

The monoclonal antibodies in the ascitic fluid were purified as follows. The frozen ascitic fluid was thawed and filtered through a nylon cloth to remove viscous material. Sufficient phenylmethyl sulfonyl fluoride was added to the ascitic fluid so that there was a final concentration of 0.1 mM. 0.05 ml of 1.2M acetate buffer (pH 4.0) was added for every milliliter of ascites fluid. The final concentration of the acetate buffer was 60 mM. The pH was adjusted to 4.5.

For every milliliter of treated ascites fluid, 25 $\mu$l of caprylic acid (MW of 144.21, density of 0.91 g/ml) was added dropwise with vigorous stirring. The suspension was kept at room temperature and stirred continuously for 30 more minutes.

The suspension was then centrifuged at 15,000 g for ten minutes in order to remove the precipitate. The supernatant, which contains IgG, was neutralized by adding a volume of 1 M HEPES buffer (pH 8.0) equal to one-tenth the volume of the supernatant. The IgG was then precipitated with 50% $(NH_4)_2SO_4$.

The precipitate was then dissolved in HEPES saline buffer. This solution was dialysed overnight against HEPES saline buffer in order to remove $(NH_4)_2SO_4$ from the IgG. The HEPES saline buffer was changed twice during the dialysis. After dialysis, the HEPES buffer saline contains purified dissolved IgG. The purified IgG was used in certain characterization assays.

Some monoclonal antibodies were purified from ascites or culture fluid by a dual column chromatography method. First, antibodies were chromatographed on DE-52 anion exchange resin (Whatman, Maidstone, England) using 0.05M Tris pH 8.0 with stepwise increments of NaCl from 0.01M to 0.15M. Antibody-containing fractions were identified by enzyme immunoassay, concentrated by Amicon filtration (Amicon, Danver, MA, YM10 membrane) and purified further on a hydroxylapatite column (Bio-Gel HT; BioRad, Richmond, CA) using a 0.01 to 0.3M phosphate buffer (pH 7.4) step gradient. Purity was assessed by isoelectric focusing (13) and SDS-PAGE using the Pharmacia PHAST system (Pharmacia, Piscatway, N.J.) and the concentration determined by $OD_{380}$nm (1.5 - 1 mg/ml).

## Example II: Characterization of the Monoclonal Antibody of the Invention

### a) Binding to Basophils Using an Immunofluorescence Assay and a Radiobinding Assay

The IgE-reactive monoclonal antibodies have been studied to determine whether they bind to basophils isolated from peripheral blood. From this second level of screening, we chose antibody to have the therapeutic value. The antibody must not bind to basophils and mast cells and cause the release of pharmacological mediators.

Intially, we chose to use immunofluorescence staining assay because basophils account for very small percentages (0.5-2%) among leukocytes. We believed that even with enriched basophil preparations, examining cells at the single cell level using immunofluorescence staining or biotin-avidin enzyme immunostaining probably gave more precise determination than radiobinding or ELISA examining the total cell populations.

### 1. Isolation of basophils

Basophils were highly enriched from peripheral blood of normal, healthy individuals using density centrifugation on Percoll by adopting a procedure described by P. Raghuprasad., J. Immunol. 129:2128-2133 (1982). Briefly, Percoll stock solution was prepared by mixing 90 ml 90% Percoll solution with 8.96 ml 10x Hanks-balanced salt solution, 0.45 ml 1N HCl, and 1 ml 10x HEPES buffer (pH 7.6). The required densities of Percoll were prepared by using the following formula (8): Percoll density (g/ml) = (% Percoll stock solution x 0.001186) + 1.0041, where 0.001186 is a constant and 1.0041 the density of physiologic media. Because the density of Percoll is altered by temperature, it is prepared before the day of experiment and kept at room temperature overnight.

Heparinized blood freshly obtained from normal donors was diluted 1:1 with basic culture medium RPM1-1640 and centrifuged on a Ficoll/Hypaque cusion (density = 1.070 g/ml). The mononuclear cells at the interface were removed for other uses and the whitish layer on top of the red cell pellets was recovered. These granulocytes were washed and resuspended in basic medium and then centrifuged through two carefully layered Percoll gradient of 1.072 and 1.078 g/ml at 600 x g for 15 minutes. The cells recovered at the interface of the Percoll layers and below the interface of basic medium/upper Percoll layer were harvested. These cells contained 2-10% of basophils, depending on the particular individual donors.

### 2. Assay Procedure

50 $\mu$l of the enriched basophil suspension at a concentration of 5 x $10^6$ cells/ml was added to each of 1.5 ml microfuge tubes containing specific antibodies. 50 $\mu$l of the supernatants from the hybridoma clones showing the greatest O.D. readings in ELISA with human IgE as the antigen was then added to each tube. With some clones repetitious assays were performed. When purified antibodies were available, they were used at 20, 5 and 1 $\mu$g/ml; when ascitic fluids were available, they were used at 1:50 dilutions.

The tubes with cells and antibodies were then incubated for 30 minutes at room temperature. After incubation, the tubes were spun, the supernatant was withdrawn, and the cells were washed two times with a mixture of RPMI 1640, containing 2% fetal calf serum and 0.1% sodium azide. The tubes were then tapped to loosen the cell pellet.

10 $\mu$l of labeled antibody, goat anti-mouse IgG conjugated with fluorescein isothiocyanate (FITC), was added to each test tube at a dilution of 1 to 200. This labeled antibody will bind to any monoclonal antibodies which have attached to IgE on basophils and provide a means for identifying these monoclonal antibodies.

The tubes were again incubated for 30 minutes at room temperature. The tubes were centrifuged, and the cells were washed with the same medium as before. The cells were then resuspended in 50 $\mu$l PBS, placed onto individual slides and cover-slipped. The cells were viewed with a fluorescence microscope.

When the antibodies stained cells, one could observe that some of cells in each viewing field were stained bright. Depending on experiments the percentages of positively stained cells range about 2-10%.

3. Radiobinding Assays

Many of the steps used are similar to those for immunofluorescence staining. The leukocyte fraction containing enriched basophils is incubated with the mouse monoclonal antibody and about 10,000 cpm of $^{125}$I-goat anti-mouse IgG in the presence of 1% normal goat serum as a blocker for nonspecific binding. After 30 minutes, the incubation mixture is then overlaid on top of calf serum (100%) in a conical plastic centrifuge tube. After centrifugation to pellet the cells, the upper layer and the serum are removed. The tubes are inverted to drain the residual liquid. The tips of the cones containing the cell pellet are then cut off with a sharp razor blade. These tips are then placed in tubes and counted for $^{125}$I in a scintillation counter. It is determined whether the binding is positive or negative by comparing the amounts of bound $^{125}$I between negative control monoclonal antibody and the human IgE-specific monoclonal antibodies.

4. Results

Initially, several experiments were performed using immunofluorescence assays. More recently, tests were repeated using similar procedure employing biotin-labeled second antibody (goat anti-mouse IgG) and peroxidase-conjugated avidin. The results from the two assays indicated that the background staining varied from one antibody to another antibody. The results also indicate that sensitivities of the assays are not better than those of histamine release assays (below). The major reason for the relatively lower sensitivities in these assays was that the percentages of basophils in the blood leukocytes from the few donors selected were all very low. Individuals with relatively high basophil percentage are preferred for these immunobinding experiments. Starting with leukocytes high in basophils, one can then prepare basophil-enriched fraction, that will be suitable for these experiments.

b) Induction of histamine release from blood leukocytes

When a monoclonal antibody specific for human IgE bind to the IgE bound on basophils, it will cross-link the IgE and aggregate the underlying Fc$\epsilon$R molecules and cause the release of histamine and other pharmacological mediators. The various human IgE-specific monoclonal antibodies were tested for ability to induce histamine release from washed human peripheral blood leukocytes.

1. Procedure

The method employed was the same as described in detail by Siraganian and Hook. The in vitro assay quantitated the percentages of total histamine in the leukocyte population that was released into the culture medium upon the incubation of inducers. The determination of histamine in the medium or cell lysates was done with an automated instrument which extracted histamine with n-butanol and reacted it with a coupling compound, o-phthaldehyde at high pH to form a fluorescent product, and measured it with fluorometer.

We performed the histamine release induction experiments, and collected media from leukocytes after the incubation with the antibodies to be tested and control antibodies, and prepared cell lysates to determine total histamine amounts.

To briefly describe the test procedure for histamine release from washed leukocytes, we adopted essentially the procedure described by Siraganian, R.P. and Hook, W.A. in Manual of Clinical Chemistry, ed. Rose, N.R. and Friedman, H., 2d Ed, pps 208-321, American Society of Microbiology, Washington, D.C. The blood was drawn from normal volunteers by venipuncture. In 50 ml conical tube, each 10 ml blood was mixed with 1 ml 0.1M EDTA and 2.5 ml dextrandextrose solution. (All solutions and reagents mentioned here are described in detail by Siraganian, supra.) The mixture was allowed to settle at room temperature for 60-90 minutes until a sharp interface developed between the erythrocyte and plasma layers. The plasma-leukocyte-platelet layer was drawn off and spun at 1,100 rpm for 8 minutes at 4°C. The supernatants containing the platelets were removed and 2-3 ml solution of cold PIPES A-EDTA was added and the cells were resuspended. Another 40 ml of cold PIPES A-EDTA was added and the cells were spun down. After the supernatants were removed, the cells were resuspended in 20 ml PIPES A. The cells were then spun down again and resuspended in PIPES ACM at cell density of $4 \times 10^6$/ml.

Tubes containing 0.3 ml of the washed leukocytes and tubes containing 0.3 ml of the culture medium of hybridomas were warmed up to 37°C in 6 minutes. The contents of the tubes were mixed and incubated at 37°C with shaking every 10 minutes. At the end of 60 minutes, the cells were spun down and the supernatants were saved. For the determination of total histamine content, 0.3 ml of the washed leukocytes were mixed with 6% perchloric acid.

2. Results

Recognizing the unreliability in the nature of the histamine release among the blood donors available to us, we contracted the histamine assay to an outside laboratory which routinely performs histamine release assays. Dr. Donald MacGlashan's laboratory in the Department of Clinical Chemistry in Johns Hopkins University determined most of our human IgE-specific monoclonal antibodies for abilities to induce histamine release from basophils. The key results relating to those monoclonal antibodies which did not induce histamine release were then confirmed at Dr. Reuben Siraganian's laboratory in National Insitute of Health. Both of these laboratories have established a stable population of high histamine releasers from screening large number of blood donors. The results of the extensive studies using leukocytes from seven donors including at least four high releasers performed by Dr. MacGlashan are summarized in Table I. The antibodies were diluted 100, 10,000, or 1,000,000 folds from ascitic fluids or purified antibodies (1 to 5 mg/ml). The histamine release data were from one representative "superreleaser". The results showed that among the 41 monoclonal antibodies, 12 did not induce histamine release.

TABLE I

Reactivity With IgE-Bearing Cells and Ability to
Induce Histamine Release by Basophils of
Mouse Monoclonal Antibodies Specific for Human IgE

| Monoclonal Antibodies | Binding to SK007 Cell Flow Cytometry | Histamine Release Antibody Dilution $1/10^2$ $1/10^4$ $1/10^6$ (% of Total Release) | | |
|---|---|---|---|---|
| **Group I** | | | | |
| E101-1 (rm, k) | + | 0 | 0 | 0 |
| E8-5-3 (rm, k) | + | 0 | 0 | 0 |
| E10-21-15 (rt, k) | + | 0 | 0 | 0 |
| E10-8-120 (rt, k) | + | 0 | 0 | 0 |
| E10-12-55 (rm, k) | + | 0 | 0 | 0 |
| E11-4-70 (rm) | + | 0 | 0 | 0 |
| **Group II** | | | | |
| E10-55-31 (rt, k) | · | 0 | 0 | 0 |
| E8-13-1 (rt, k) | · | 0 | 0 | 0 |
| E8-32-9 (rt, k) | · | 0 | 0 | 0 |
| E357-4 (rt, k) | · | 0 | 0 | 0 |
| E8-37-4 (rt, k) | · | 0 | 0 | 0 |
| E8-4-17 (rt, k) | · | 0 | 0 | 0 |
| **Group III** | | | | |
| E69-2 (rt, k) | + | 44 | 13 | 0 |
| E10-100-9 (rm, k) | + | 68 | 45 | 0 |
| E10-41-16 (rm) | + | 95 | 91 | 91 |
| E10-95-3 (rm, k) | + | 65 | 68 | 3 |
| E10-68-10 (rt, k) | + | 56 | 42 | 2 |
| E10-10-3 (rt, k) | + | 85 | 78 | 2 |
| E10-5-83 (rt, k) | + | 82 | 80 | 6 |
| E10-24-28 (rm, k) | + | 79 | 89 | 5 |
| E10-27-5 (rm, k) | + | 68 | 90 | 9 |
| E10-22-84 (rm, k) | + | 77 | 84 | 15 |
| E10-74-28 (rt, k) | + | 60 | 65 | 3 |
| E10-1-88 (rt, k) | + | 72 | 55 | 2 |
| E10-3-14-25 (rt, k) | + | 68 | 58 | 3 |
| E10-7-19 (rt, k) | ÷ | 38 | 26 | 6 |
| E10-18-3 (rt, k) | + | 33 | 26 | 0 |
| E10-40-62 (rm, k) | + | 37 | 24 | 9 |
| E10-19-12 (rt, k) | + | 28 | 37 | 36 |

23

| Monoclonal Antibodies | Binding to SK007 Cell Flow Cytometry | Histamine Release Antibody Dilution $1/10^2$ $1/10^4$ $1/10^6$ (% of Total Release) | | |
|---|---|---|---|---|
| E10-52-38 (n, k) | + | 57 | 64 | 14 |
| E10-54-26 (n, k) | + | 34 | 17 | 2 |
| E235-6 (n, k) | + | 52 | 25 | 12 |
| E10-14-52 (n, k) | + | 52 | 24 | 0 |
| E10-71-47 (n, k) | + | 30 | 16 | 0 |
| E10-25-44 (n, k) | + | 31 | 26 | 0 |
| E10-61-6 (n, k) | + | 81 | 78 | 70 |
| E10-33-22 (n) | + | 77 | 74 | 16 |
| E608-10 (n) | + | 81 | 84 | 86 |
| E688-13 (n) | + | 86 | 91 | 90 |
| E10-80-4 (n) | + | 85 | 92 | 90 |
| E545-4 (n, k) | + | 24 | 3 | 0 |

c) Binding of monoclonal antibodies to IgE-secreting Myeloma Cells

Some myeloma cells (which are tumor cells derived from immunoglobulin-secreting plasma cells) are known to express low levels of immunoglobulins on their surface, compared to those on the surface of resting B cells. IgE molecules are bound to the surfaces of basophils (or mast cells) and B cells by two different mechanisms. IgE binds to basophils and mast cells via the interaction of FcεR molecules on these cells and a certain site on the Fc of IgE. IgE are synthesized by B cells or plasma cells and are transported to the cell surface and retained on the surface by an extra constant heavy chain segment. This anchoring segment is found only in membrane-bound immunoglobulins and not in secreted forms of immunoglobulins. The differential binding of a monoclonal antibody to IgE on basophils and on B cells is a fundamental basis for the application of the antibodies for therapy of allergy.

Since IgE-bearing B cells and plasma cells are very few in the mononuclear leukocyte fraction and since the topographical and structural characteristics of membrane-bound IgE molecules are most likely the same on plasma cells, B cells or IgE-secreting myeloma cells, we have chosen to study the binding of monoclonal antibodies to IgE myeloma SK007 (from American Type Culture Collection) cells. The interaction of the monoclonal antibodies can also be examined with normal IgE-bearing B cells and plasma cells.

1. Procedure

Human SKO-007, CCL-156 and CCL-159 cells, expressing surface-bound human IgE-lambda, IgM-lambda and IgG1-kappa, respectively, were maintained in RPMI medium 1640 supplemented with 5% fetal bovine serum and 2mM glutamine from GIBCO and 1% antibiotic-antimycotic solution. Peripheral blood mononuclear cells obtained by venipuncture of healthy donors were prepared by Ficoll-Hypaque (Pharmacia, Piscataway, N.J.) density gradient centrifugation. Binding of monoclonal antibodies to cell surfaces was assessed using two types of assays: binding of antibodies to live cells followed by indirect fluorescent flow cytometric analysis and an enzyme-linked antibody assay with the cells attached to microtiter plates.

Binding of antibodies to live cells was performed by pelleting the cells by centrifugation at 300xg for 5 min. washing maintenance media from the cells with PBS-BSA and resuspending the cells at $20 \times 10^6$ in PBS-B. Fifty $\mu$l of cell suspension was mixed with 50 $\mu$l of antibody at twice the stated concentrations (1-10 $\mu$g/ml) in PBS-B and kept on ice. After a 30 min incubation, 2 ml of ice cold PBS-B was added to each tube, and the cells were collected by centrifugation at 300xg for 5 min at 5°C. Supernatant was decanted, cell pellets resuspended by vortexing and cells washed once with an additional 2 ml of PBS-B. After collecting the cells by centrifugation, 20 $\mu$l of affinity purified goat F(ab')₂ anti-mouse IgG (H+L) (Boehringer Mannheim, Lot 52934, code 60529) diluted 1:20 in PBS-B was added to each tube. The tubes were incubated 20 min on ice and washed with PBS-B as above. Finally, cell pellets were resuspended in 0.5 ml of 1% paraformaldehyde (Polysciences, Inc., Warrington, PA) in PBS. Cells were analyzed using a EPICS Profile (Coulter, Hialeah, FL) equipped with a 5W argon laser running at 488 nm, 0.6W at Cytology Technology, Inc. (Houston, TX). Fluorescence intensity was collected with a built-in logarithmic amplifier after gating on the combination of forward light scatter and perpendicular light scatter to discriminate viable cells.

The cell-bound enzyme-linked antibody assay was performed by binding MAbs to glutaraldehyde-fixed cells according to the method of Kennett, R.H. in Monoclonal Antibodies, eds. Kennett, R.H. et al., pp. 376, Plenum Press, New York (1980). Poly-L-lysine (100 $\mu$l/well, 10 $\mu$g/ml in PBS) was added to flat bottomed microtiter plates (Falcon #3072, Becton Dickinson Labware, Oxnard, CA). This solution was flicked out of the wells after 30 min at 22°C and 50 $\mu$l of cells at $2.5 \times 10^6$ cells/ml of calcium and magnesium-free Dulbecco-modified PBS (GIBCO) was added to each well. Cells were deposited on the bottom of the wells by centrifugation at 300xg for 5 min and cells were fixed at 22°C for 10 min by adding 50 $\mu$l of glutaraldehyde diluted to 0.25% in ice-cold PBS. Non-specific binding sites were blocked by sequential incubation of 0.1M glycine-0.1%BSA in PBS (200 $\mu$l/well) followed by Blotto [5% non-fat dry milk (Carnation, LA, CA) in PBS with 1 g/L of thimerosal]. Blocking solutions were removed by gentle flicking of the plate. Cells were exposed to 50 $\mu$l/well of control or test monoclonal antibody in Blotto for 1 hr at 37°C. Unbound antibody was removed by flicking the plate and washing 6 times with 200 $\mu$l/well of PBS using a Transtar 96 pipetting device (Costar, Cambridge, MA). Subsequently, the cells were incubated with 50 ul biotin-labeled affinity-purified goat anti-mouse IgG(KPL, Gaithersburg, MD) at 0.5 $\mu$g/ml in Blotto for 1 hr at 37°C. All wells were washed as above and horseradish peroxidase-streptavidin was added at 0.5 $\mu$g/ml in Blotto for 1 hr at 37°C. Unbound conjugate was removed by washing as above and 100 $\mu$l of TMB substrate was added. The plates were kept in the dark for 30 min at 22°C and the reaction was stopped with the addition of 50 $\mu$l/well of 4N $H_2SO_4$. Optical density at 450 nm was measured using a Biotek microtiter plate reader.

2. Results

Among the 41 monoclonal antibodies tested with flow cytometric analyses, 35 were shown to stain SK007 cells. All of the 29 monoclonal antibodies which induced histamine release from basophils, stained. Among the 12 monoclonal antibodies, which did not induce histamine release, 6 stained and 6 did not stain SK007 cells (Table I). The results with enzyme immunostaining were the same as those with flow cytometric assays. Thus, in the group of monoclonal antibodies that have been analyzed, six fit the criteria that they do not bind to basophils and induce histamine release and that they bind to IgE-producing B cells. As discussed earlier, Siraganian and his colleagues (Fed. Proc. 46:1346, (1987)) developed two mouse monoclonal antibodies (E14C5IB1 and E11AC3IIC) against human IgE that could inhibit binding of IgE to basophils. We obtained these two antibodies from Dr. Siraganian and showed that they bind to SK007 cells and did not induce histamine release from basophils.

d) Determining the Binding Affinity with Human IgE

1. Principle and Procedure

It is well known that the sensitivity of immunoassays depends on the affinities of the antibodies for the substances to be measured. In the cases of solid phase sandwich immunoassays using two monoclonal antibodies, one as the solid-phase adsorbent and one as the tracer, both of the affinities of the two monoclonal antibodies for the antigen are important. The influence of antibody affinity on the performance of different antibody assays and the use of immunoassays for calculating antibody affinity have been systematically studied. Nimmo et al. J. Immunol. Met. 72:177-187 (1984); Muller J. Immunol. Met. 34:345-352 (1980).

For determining the affinity of a monoclonal antibody for an antigen, one can coat the antigen on the solid phase of an immunoassay, for example, the microtiter wells of a 96-well ELISA plate. The affinity of a monoclonal antibody relative to that of a reference monoclonal antibody for the same antigen on the solid phase can be determined by comparing the two monoclonal antibodies in the immunoassay. The affinity or the association constant of the reference monoclonal antibody has been determined by a certain other method or a similar method. The O.D. readout of the monoclonal antibody which affinity is to be determined in comparison to that of the reference monoclonal antibody will indicate whether the affinity of that monoclonal antibody is greater or lower than that of the reference monoclonal antibody.

25

When a reference monoclonal antibody is not available, the analysis can be made against a reference monoclonal antibody specific for a different antigen. By coating same molar amount antigen on the solid phase and applying all other assay conditions and parameters identical, the relative affinity of the two monoclonal antibodies can be determined from the O.D. readouts.

In our determination of the affinities of the several human IgE-specific monoclonal antibodies that bind to SK007 cells but do not induce histamine release from basophils, we compared the binding of various monoclonal antibodies to human IgE with that of a monoclonal antibody to human $\beta$-HCG, which affinity has been determined to be $1 \times 10^{11}$ liter/mole. In our assays, we coated $50\mu$l of 0.1 $\mu$g/ml of $\beta$-HCG or human IgE on the wells of an ELISA plate and titrated the anti-HCG monoclonal antibody and the various monoclonal antibodies against the respective antigens on the solid phase. The procedure was in effect the same as described in the ELISA procedure in Example I. By using horseradish peroxidase-conjugated goat-anti-mouse IgG and the enzyme substrate, the titration curves were determined.

The affinity of monoclonal antibodies of interest can also be determined by $^{125}$I-labeled human IgE. The solutions of the antibodies and $^{125}$I-IgE of known concentrations are mixed and the mixture is allowed sufficient time (24 hours) for the binding to reach to equilibrium. The immune complexes are then swiftly removed by affinity adsorption using excess Sepharose 4B conjugated with goat-anti-mouse IgG. The free $^{125}$I-IgE is washed off swiftly. From the proportions of free $^{125}$I-IgE and bound $^{125}$I-IgE, the association constant, Ka, of the monoclonal antibody can be calculated. This method is especially suitable for antibodies of high affinity.

2. Results

The six monoclonal antibodies that do not induce histamine release from basophils and that bind to SK007 cells have been determined to have association constant, Ka, in the range of $3 \times 10^8$ to $5 \times 10^9$ liter/mole.

Equivalents
‾ ‾ ‾ ‾ ‾ ‾

Those skilled in the art will recognize, or be able to ascertain using no more than routine experimentation, many equivalents to the specific embodiments of the inventions described herein. Such equivalents are intended to be encompassed by the following.

**Claims**

1. A peptide having an amino acid sequence substantially equivalent to the extracellular segment of the membrane-bound domain of IgE.

2. Oligonucleotides coding for the peptide of claim 1.

3. The oligonucleotides of claim 2 which are ribonucleic acid or deoxyribonucleic acid.

ESTABLISHING cDNA LIBRARY FROM mRNA
ISOLATED FROM A HUMAN IgE-BEARING MYELOMA

SCREENING CLONES ENCOMPASSING THE
MEMBRANE BOUND SEGMENT OF ε IMMUNOGLOBULIN

SEQUENCING THE ε MEMBRANE BOUND SEGMENT

DETERMINING THE mb/ec PORTION FROM THE
COMPARISON WITH THE KNOWN SEQUENCE
OF ε CHAIN OF SECRETORY IgE AND MEMBRANE
BOUND SEGMENTS OF IMMUNOGLOBULIN
HEAVY CHAINS

PROCESS OF DETERMINING THE DNA SEQUENCE
OF mb/ec SEGMENT OF HUMAN ε CHAIN (ε.mb/ec)
USING cDNA CLONING APPROACH

FIG. I

CH4 DOMAIN

|  |  |  |  |  | PROBE d |  |  |  |  |  |  |  |  |
| TCC | CGC | TTC | TTC | GTC | TTC | AGC | CGC | CTG | GAG | GTG | ACC | AGG | GCC | GAA |
| Ser | Gly | Phe | Phe | Val | Phe | Ser | Arg | Leu | Glu | Val | Thr | Arg | Ala | Glu |

SPLICING DONOR

| TGG | GAG | CAG | AAA | GAT | GAG | TTC | ATC | TGC | CGT | GCA | GTC | CAT | GAG | GCA | GCG | AGC | CCC | TCA | GAG |
| Trp | Glu | Gln | Lys | Asp | Glu | Phe | Ile | Cys | Arg | Ala | Val | His | Glu | Ala | Ala | Ser | Pro | Ser | Gln |

SPLICING DONOR PROBE b

| ACC | GTC | CAG | CGA | GCG | GTG | TCT | GTA | AAT | CCC | GGT | AAA | TGA |
| Thr | Val | Gln | Arg | Ala | Val | Ser | Val | Asn | Pro | Gly | Lys | End |

FIG. 3

SECRETED IgE

CH4

PROBE a          PROBE b

PROBE d

PROBE c

TRUNCATED CH4          mb/ec          mb/tm          mb/ic

MEMBRANE-BOUND REGION

MEMBRANE-BOUND IgE

FIG. 2

EP 0 617 127 A1

## DOCUMENTS CONSIDERED TO BE RELEVANT

| Category | Citation of document with indication, where appropriate, of relevant passages | Relevant to claim | CLASSIFICATION OF THE APPLICATION (Int.Cl.4) |
|---|---|---|---|
| A | US-A-4 714 759 (WHITACKER, JR) <br> * claims * <br> --- | 1-3 | C12N15/13 <br> C07K15/00 <br> C12P21/08 |
| A | EP-A-0 205 405 (CIBA-GEIGY AG) <br> * claims * <br> ----- | 1-3 | A61K39/395 <br> C12N15/06 <br> G01N33/577 |

|  |
|---|
| TECHNICAL FIELDS SEARCHED (Int.Cl.4) |
| C12N <br> C07K <br> C12P <br> A61K <br> G01N |

The present search report has been drawn up for all claims

| Place of search | Date of completion of the search | Examiner |
|---|---|---|
| THE HAGUE | 6 June 1994 | Nooij, F |

EPO FORM 1503 03.82 (P04C01)